(19) Europäisches Patentamt
European Patent Office
Office européen des brevets

(11) Veröffentlichungsnummer: **0 333 059**
**A2**

(12) # EUROPÄISCHE PATENTANMELDUNG

(21) Anmeldenummer: **89104266.5**

(22) Anmeldetag: **10.03.89**

(51) Int. Cl.⁴: **C07D 249/08 , C07D 233/60 , A01N 43/653 , A01N 43/50**

(30) Priorität: **18.03.88 DE 3809069**

(43) Veröffentlichungstag der Anmeldung: **20.09.89 Patentblatt 89/38**

(84) Benannte Vertragsstaaten: **AT BE CH DE ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Seele, Rainer, Dr.**
**Leiblstrasse 3**
**D-6701 Fussgoenheim(DE)**
Erfinder: **Sauter, Hubert, Dr.**
**Neckarpromenade 20**
**D-6800 Mannheim 1(DE)**
Erfinder: **Kober, Reiner, Dr.**
**Im Schlittweg 20**
**D-6701 Fussgoenheim(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**

(54) **Azolylmethylallylalkohole und diese enthaltende Fungizide.**

(57) Azolylmethylallylalkohole der allgemeinen Formel I

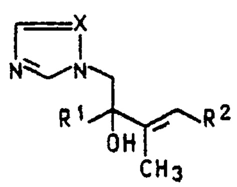

(I)

in welcher $R_1$ und $R_2$ Alkyl, Naphthyl, Biphenyl, Cycloalkyl, Cycloalkenyl oder Phenyl bedeuten, wobei diese Reste gegebenenfalls substituiert sind,
X, CH oder N bedeutet, sowie deren für Pflanzen verträgliche Säureadditionssalze und Metallkomplexe und diese Verbindungen enthaltende Fungizide.

EP 0 333 059 A2

Xerox Copy Centre

## Azolylmethylallylalkohole und diese enthaltende Fungizide

Die vorliegende Erfindung betrifft neue Azolverbindungen, Verfahren zu ihrer Herstellung und diese enthaltende Fungizide.

Es ist bekannt, Triazolyl-butenol-Derivate, z.B. das 1-(1,2,4-Triazol-1-yl-methyl)-1-(4-chlorphenyl)-3-(2-chlorphenyl)-prop-2-en-1-ol als Fungizide zu verwenden (EP-52 424). Seine fungizide Wirkung ist jedoch ungenügend.

Es wurde nun gefunden, daß Azolylmethylallylalkohole der allgemeinen Formel I

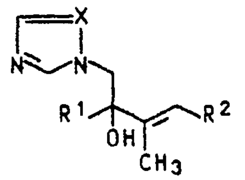

(I)

in welcher $R_1$ und $R_2$ gleich oder verschieden sind und $C_1$-$C_4$-Alkyl, Naphthyl, Biphenyl, $C_3$-$C_{12}$-Cycloalkyl, Cycloalkenyl oder Phenyl bedeuten, wobei diese Reste gegebenenfalls einfach bis dreifach durch Halogen, Nitro, Phenoxy, Alkyl, Alkoxy, Amino oder Halogenalkyl mit jeweils 1 bis 4-C-Atomen substituiert sind, X CH oder N bedeutet, sowie deren für Pflanzen verträgliche Säureadditionssalze und Metallkomplexe eine bessere fungizide Wirkung haben, insbesondere gegen Getreidekrankheiten, als die bekannte Azolverbindung.

Die Verbindungen der Formel I werden im allgemeinen in Form von Racematen mit E-Konfiguration an der C = C-Doppelbindung erhalten. Die Racemate lassen sich mit bekannten Methoden, beispielsweise über diastereomere Ester von optisch reinen Säuren trennen und in reiner Form isolieren. Als fungizide Mittel kann man sowohl die einheitlichen Enantiomere wie auch deren bei der Synthese anfallenden Racemate verwenden. Die vorliegende Erfindung umfaßt alle diese Verbindungen.

$R^1$ oder $R^2$ bedeuten beispielsweise: Methyl, Ethyl, Isopropyl, n-Propyl, n-Butyl, sec.-Butyl, tert.-Butyl, Phenyl, Halogenphenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, Alkoxyphenyl, 2-Methoxyphenyl, 3-Methoxyphenyl, 4-Methoxyphenyl, 2,4-Dimethoxyphenyl, Alkylphenyl, 4-Ethylphenyl, 4-Isopropylphenyl, 4-tert.- Butylphenyl, 4-tert.-Butyloxyphenyl, 2-Chlor-4-fluorphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, 2-Trifluoromethylphenyl, 3-Trifluoromethylphenyl, 4-Trifluoromethylphenyl, Cyclopropyl, Cyclobutyl, Cyclopentyl, Cyclohexyl, Cyclooctyl, Cyclododecyl, 2-Cyclohexenyl, 3-Cyclohexenyl, Naphthyl, Biphenyl.

Säureadditionssalze sind beispielsweise die Salze mit anorganischen oder organischen Säuren, z.B. die Hydrochloride, Bromide, Sulfate, Nitrate, Phosphate, Oxalate oder Dodecylbenzolsulfonate. Die Wirksamkeit der Salze geht auf das Kation zurück, so daß das Anion i.a. beliebig gewählt werden kann. Die erfindungsgemäßen Wirkstoffsalze werden hergestellt durch Umsetzung der Azolylmethyloxirane mit den Säuren.

Metallkomplexe der Wirkstoffe I oder ihrer Salze können z.B. mit den Metallen Kupfer, Zink, Zinn, Mangan, Eisen, Kobalt oder Nickel gebildet werden, indem man die Azolylmethylallylalkohole mit den Metallsalzen umsetzt, z.B. mit Kupfersulfat, Zinnchlorid, Zinksulfat.

Die Verbindungen der Formel I können z.B. hergestellt werden, indem man eine Verbindung der Formel II

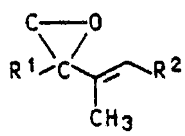

II

in welcher $R_1$ und $R_2$ die oben angegebenen Bedeutungen haben, mit einer Verbindung der Formel III

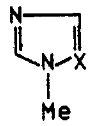

III

in der Me ein Wasserstoffatom oder ein Metallatom (Na, K) bedeutet und X die angegebene Bedeutung hat, umsetzt.

Die Reaktion erfolgt - falls Me ein Wasserstoffatom bedeutet - gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120 ° C. Zu den bevorzugten Lösungs- und Verdünnungsmitteln gehören Ketone wie Aceton, Methylethylketon oder Cyclohexanon. Nirile wie Acetonitril oder Propionitril, Alkohole wie Methanol, Ethanol, iso-Propanol, n-Butanol oder Glycol, Ester wie Essigsäureethylester, Essigsäuremethyl ester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon, ferner Dimethylsulfoxid, Sulfolan oder entsprechende Gemische.

Geeignete Basen, die gegebenenfalls auch als säurebindende Mittel bei der Reaktion verwendet werden können, sind beispielsweise Alkalihydroxid wie Lithium-, Natrium- oder Kaliumhydroxid, Alkalicarbonate wie Natrium-, Kalium- oder Caesiumcarbonat oder Natrium-, Kalium- oder Caesiumhydrogencarbonat, Pyridin oder 4-Dimethylaminopyridin. Es können aber auch andere übliche Basen verwendet werden.

Als Reaktionsbeschleuniger kommen vorzugsweise Metallhalogenide wie Natriumiodid, oder Kaliumiodid, quarternäre Ammoniumsalze wie Tetrabutylammoniumchlorid, -bromid, -jodid oder -hydrogensulfat, Benzyltriethylammoniumchlorid oder -bromid oder Kronenether wie 12-Krone-4, 15-Krone-5, 18-Krone-6, Dibenzo-18-Krone-6 oder Dicyclohexano-18-Krone-6 in Frage.

Die Umsetzung wird im allgemeinen bei Temperaturen zwischen 20 und 150 ° C, drucklos oder unter Druck, kontinuierlich oder diskontinuierlich durchgeführt.

Ist Me ein Metallatom wird die Reaktion a) gegebenenfalls in Gegenwart eines Lösungs- oder Verdünnungsmittels und gegebenenfalls unter Zusatz einer starken anorganischen oder organischen Base bei Temperaturen zwischen -10 und 120 ° C durchgeführt. Zu den bevorzugten Lösungs- und Verdünnungsmitteln gehören Amide wie Dimethylformamid, Diethylformamid, Dimethylacetamid, Diethylacetamid, N-Methylpyrrolidon, Hexamethyl-phosphorsäuretriamid, Sulfoxide wie Dimethylsulfoxid und Sulfolan.

Geeignete Basen, die gegebenenfalls auch als säurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Natrium- oder Kalium-tert.-butoxid, Lithium-, Natrium- oder Kalium-triphenylmethyl und Naphthalinlithium, -natrium oder -kalium.

Für die Reaktion b) kommen als Verdünnungsmittel polare organische Lösungsmittel wie Nitrile, z.B. Acetonitril, Sulfoxide, z.B. Dimethylsulfoxid, Formamide, z.B. Dimethylformamid, Ketone, z.B. Aceton, Ether, z.B. Diethylether, Tetrahydrofuran und insbesondere Chlorkohlenwasserstoffe, z.B. Methylenchlorid und Chloroform, in Frage.

Man arbeitet im allgemeinen zwischen 0 und 100 ° C, vorzugsweise bei 20 bis 80 ° C. Bei Anwesenheit eines Lösungsmittels wird zweckmäßigerweise beim Siedepunkt des jeweiligen Lösungsmittels gearbeitet.

Die neuen Ausgangsverbindungen II lassen sich nach bekannten Methoden in einfacher Weise aus den ungesättigten Ketonen der Formel IV herstellen

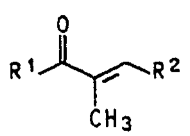

IV

(vgl. Corey, Chaykovsky, J. Am. Chem. Soc. 1962, 64, 3782)

Die Verbindungen IV lassen sich entsprechend allgemein bekannten Verfahren zur Olefinsynthese (Houben-Weyl-Müller, Methoden der organischen Chemie, Georg Thieme Verlag, Stuttgart, 1972 Bd. V, 1b) herstellen.

Die folgenden Beispiele erläutern die Herstellung der Wirkstoffe.

I. Herstellung der Ausgangsstoffe

Beispiel A

Zu einer Lösung von 50 g 2-Chlorbenzaldehyd in 200 ml Ethanol werden 5,85 g Natriumhydroxid in 40 ml Wasser gegeben. Das Reaktionsgemisch wird auf 10 °C gekühlt und 60 g 4-Chlorpropiophenon zugesetzt, wobei die Temperatur in der Lösung auf 30 bis 40 °C ansteigt. Nach 10stündigem Rühren bei 50 °C wird der Reaktionslösung 200 ml Wasser zugesetzt und die entstandene Emulsion mit Methyl-tert.-butylether ausgeschüttelt. Die organische Phase wird abgetrennt, über Natriumsulfat getrocknet und im Wasserstrahlvakuum eingeengt. Bei der anschließenden Destillation des verbleibenden Rückstandes werden bei 0,25 mbar und 127 °C Übergangstemperatur 84 g (80 %) 4-Chlorphenyl-β-methyl-2-chlorphenylstyrylketon erhalten, das aus Methyl-tert.-butylether/n-Hexan kristallisiert werden kann, Fp.: 45 -47 °C.

Beispiel B

Zu einer Lösung von 84 g 4-Chlorphenyl-β-methyl-2-chlorphenylstyrylketon in 300 ml Methylenchlorid werden 54 g Trimethylsulfoniummethylsulfat und 120 ml Natronlauge (50 gew.%ig) gegeben. Nachdem das Reaktionsgemisch 12 -15 Stunden bei Raumtemperatur (20 °C) gerührt worden ist, wird der Lösung 300 ml Wasser zugesetzt und die organische Phase abgetrennt. Die verbleibende organische Phase wird zweimal mit Wasser gewaschen. Die isolierte organische Phase wird über Natriumsulfat getrocknet und eingeengt, wobei 73 g (83 %) 2-(4-Chlorphenyl)-2-(1-methyl-2-[2-chlorphenyl]-ethenyl)-oxiran erhalten wird.

II. Herstellung der Endprodukte

Beispiel 1

Eine Lösung von 70 g Triazol in 300 ml N-Methyl-pyrrolidon wird mit 12 g Natriumhydroxid versetzt und für 30 Minuten auf 50 °C erhitzt. Anschließend wird bei Raumtemperatur 73 g 2-(4-Chlorphenyl)-2-(1-methyl-2-[2-chlorphenyl]-ethenyl)-oxiran, das in 100 ml N-Methyl-pyrrolidon gelöst ist, langsam zugetropft. Nachdem das Reaktionsgemisch 15 Stunden bei Raumtemperatur gerührt worden ist, wird der Lösung 300 ml Wasser zugesetzt und mehrmals mit Methyl-tert.-butylether ausgeschüttelt. Die isolierte organische Phase wird zweimal mit Wasser gewaschen, daraufhin über Natriumsulfat getrocknet und eingeengt. Durch Kristallisation des Rückstandes aus Methyl-tert.-butylether/n-Hexan erhält man 85 g (95 %) 1-(1,2,4-Triazol-1-yl-methyl)-1-(4-chlorphenyl)-2-methyl-3-(2-chlorphenyl)-propenol mit einem Schmelzpunkt von 141 - 143 °C.

Entsprechend Beispiel 1 können die in der Tabelle aufgeführten Verbindungen hergestellt werden.

i Tabelle

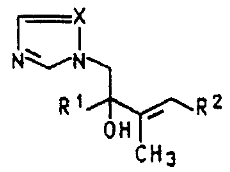

| Beispiel | $R_1$ | $R_2$ | X | Schmp./IR (°C) |
|---|---|---|---|---|
| 1 | 4-Cl-$C_6H_4$ | 2-Cl-$C_6H_4$ | N | 141 - 143 |
| 2 | 4-Cl-$C_6H_4$ | 2-Cl-$C_6H_4$ | CH | |
| 3 | Phenyl | 3-Cl-$C_6H_4$ | N | |
| 4 | Phenyl | 3-Cl-$C_6H_4$ | CH | |
| 5 | Phenyl | 4-Cl-$C_6H_4$ | N | |
| 6 | Phenyl | 4-Cl-$C_6H_4$ | CH | |
| 7 | Phenyl | 2,4-$Cl_2$-$C_6H_3$ | N | 153 - 157 |
| 8 | Phenyl | 2,4-$Cl_2$-$C_6H_3$ | CH | |

| Beispiel | $R_1$ | $R_2$ | X | Schmp./IR (°C) |
|---|---|---|---|---|
| 9 | Phenyl | $2\text{-}F\text{-}C_6H_4$ | N | 162 - 164 |
| 10 | Phenyl | $2\text{-}F\text{-}C_6H_4$ | CH | 182 - 184 |
| 11 | Phenyl | $4\text{-}F\text{-}C_6H_4$ | N | 145 - 147 |
| 12 | Phenyl | $4\text{-}F\text{-}C_6H_4$ | CH | 164 - 166 |
| 13 | Phenyl | $2\text{-}Cl\text{-}4\text{-}F\text{-}C_6H_3$ | N | |
| 14 | Phenyl | $2\text{-}Cl\text{-}4\text{-}F\text{-}C_6H_3$ | CH | |
| 15 | Phenyl | $3\text{-}NO_2\text{-}C_6H_4$ | N | |
| 16 | Phenyl | $4\text{-}NO_2\text{-}C_6H_4$ | N | |
| 17 | Phenyl | $3\text{-}NH_2\text{-}C_6H_4$ | N | |
| 18 | Phenyl | $4\text{-}NH_2\text{-}C_6H_4$ | N | |
| 19 | Phenyl | $2\text{-}OCH_3\text{-}C_6H_4$ | N | |
| 20 | Phenyl | $4\text{-}OCH_3\text{-}C_6H_4$ | N | |
| 21 | Phenyl | 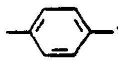tert.Butyl | N | |
| 22 | Phenyl | Cyclohexyl | N | |
| 23 | Phenyl | Cyclohexyl | CH | |
| 24 | Phenyl | Cyclohexenyl | N | |
| 25 | Phenyl | Cyclohexenyl | CH | |
| 26 | Phenyl | Cyclopentyl | CH | |
| 27 | Phenyl | Cyclopentyl | N | |
| 28 | Phenyl | Cyclopropyl | CH | |
| 29 | Phenyl | Phenyl | N | 168 - 170 |
| 30 | Phenyl | Norbornyl | N | |
| 31 | Phenyl | Norbornyl | CH | |
| 32 | Phenyl | $2\text{-}Cl\text{-}C_6H_4$ | N | 130 - 132 |
| 33 | Phenyl | $2\text{-}Cl\text{-}C_6H_4$ | CH | 155 - 157 |
| 34 | $4\text{-}Cl\text{-}C_6H_4$ | $4\text{-}Cl\text{-}C_6H_4$ | N | 1490, 1276, 1092, 1014, 831 $cm^{-1}$ |
| 35 | $4\text{-}Cl\text{-}C_6H_4$ | $4\text{-}Cl\text{-}C_6H_4$ | CH | |
| 36 | $4\text{-}Cl\text{-}C_6H_4$ | $3\text{-}Cl\text{-}C_6H_4$ | N | |
| 37 | $4\text{-}Cl\text{-}C_6H_4$ | $3\text{-}Cl\text{-}C_6H_4$ | CH | |
| 38 | $4\text{-}Cl\text{-}C_6H_4$ | $2,4\text{-}Cl_2\text{-}C_6H_3$ | N | 164 - 166 |
| 39 | $4\text{-}Cl\text{-}C_6H_4$ | $2,4\text{-}Cl_2\text{-}C_6H_3$ | CH | 1469, 1276, 1087, 860 $cm^{-1}$ |
| 40 | $4\text{-}Cl\text{-}C_6H_4$ | Phenyl | N | |
| 41 | $4\text{-}Cl\text{-}C_6H_4$ | Phenyl | CH | |
| 42 | $4\text{-}Cl\text{-}C_6H_4$ | $2\text{-}F\text{-}C_6H_4$ | N | |
| 43 | $4\text{-}Cl\text{-}C_6H_4$ | $2\text{-}F\text{-}C_6H_4$ | CH | |
| 44 | $4\text{-}Cl\text{-}C_6H_4$ | $4\text{-}F\text{-}C_6H_4$ | N | Harz |
| 45 | $4\text{-}Cl\text{-}C_6H_4$ | $4\text{-}F\text{-}C_6H_4$ | CH | 174-176°C |

| Beispiel | $R_1$ | $R_2$ | X | Schmp./IR (°C) |
|---|---|---|---|---|
| 46 | $4-Cl-C_6H_4$ | $3-NO_2-C_6H_4$ | N | |
| 47 | $4-Cl-C_6H_4$ | $4-NO_2-C_6H_4$ | N | |
| 48 | $4-Cl-C_6H_4$ | $2-Cl-4-F-C_6H_3$ | N | |
| 49 | $4-Cl-C_6H_4$ | $2-Cl-4-F-C_6H_3$ | CH | |
| 50 | $4-Cl-C_6H_4$ | $4-NH_2-C_6H_4$ | N | |
| 51 | $4-Cl-C_6H_4$ | $3-NH_2-C_6H_4$ | CH | |
| 52 | $4-Cl-C_6H_4$ | $2-OCH_3-C_6H_4$ | N | |
| 53 | $4-Cl-C_6H_4$ | $4-OCH_3-C_6H_4$ | N | |
| 54 | $4-Cl-C_6H_4$ | Cyclohexyl | N | |
| 55 | $4-Cl-C_6H_4$ | Cyclohexyl | CH | |
| 56 | $4-Cl-C_6H_4$ | Cyclohexenyl | N | |
| 57 | $4-Cl-C_6H_4$ | Cyclopentyl | N | |
| 58 | $2-Cl-C_6H_4$ | Phenyl | N | |
| 59 | $2-Cl-C_6H_4$ | $2-Cl-C_6H_4$ | N | |
| 60 | $2-Cl-C_6H_4$ | $3-Cl-C_6H_4$ | N | |
| 61 | $2-Cl-C_6H_4$ | $4-Cl-C_6H_4$ | N | |
| 62 | $2-Cl-C_6H_4$ | $2,4-Cl_2-C_6H_3$ | N | |
| 63 | $2-Cl-C_6H_4$ | $2-F-C_6H_4$ | N | |
| 64 | $2-Cl-C_6H_4$ | $4-F-C_6H_4$ | N | |
| 65 | $2-Cl-C_6H_4$ | $2-Cl-4-F-C_6H_3$ | N | |
| 66 | $2-Cl-C_6H_4$ | $3-NO_2-C_6H_4$ | N | |
| 67 | $2-Cl-C_6H_4$ | $4-NO_2-C_6H_4$ | N | |
| 68 | $2-Cl-C_6H_4$ | $3-NH_2-C_6H_4$ | N | |
| 69 | $2-Cl-C_6H_4$ | $4-NH_2-C_6H_4$ | N | |
| 70 | $2-Cl-C_6H_4$ | $2-OCH_3-C_6H_4$ | N | |
| 71 | $2-Cl-C_6H_4$ | $4-OCH_3-C_6H_4$ | N | |
| 72 | $2-Cl-C_6H_4$ | Cyclohexyl | N | |
| 73 | $2-Cl-C_6H_4$ | Cyclohexenyl | N | |
| 74 | $2-Cl-C_6H_4$ | Cyclopentyl | N | |
| 75 | $2-Cl-C_6H_4$ | tert.-butyl | N | |
| 76 | $2-Cl-C_6H_4$ | norbornyl | N | |
| 77 | $2-Cl-C_6H_4$ | iso-propyl | N | |
| 78 | $2-F-C_6H_4$ | Phenyl | N | |
| 79 | $2-F-C_6H_4$ | $2-Cl-C_6H_4$ | N | |
| 80 | $2-F-C_6H_4$ | $3-Cl-C_6H_4$ | N | |
| 81 | $2-F-C_6H_4$ | $4-Cl-C_6H_4$ | N | |
| 82 | $2-F-C_6H_4$ | $2,4-Cl_2-C_6H_3$ | N | |
| 83 | $2-F-C_6H_4$ | $2-F-C_6H_4$ | N | |
| 84 | $2-F-C_6H_4$ | $4-F-C_6H_4$ | N | |
| 85 | $2-F-C_6H_4$ | $4-NO_2-C_6H_4$ | N | |

| Beispiel | $R_1$ | $R_2$ | X | Schmp./IR (°C) |
|---|---|---|---|---|
| 86 | $2-F-C_6H_4$ | $3-NH_2-C_6H_4$ | N | |
| 87 | $2-F-C_6H_4$ | $2-OCH_3-C_6H_4$ | N | |
| 88 | $2-F-C_6H_4$ | Cyclohexyl | N | |
| 89 | $4-F-C_6H_4$ | $2-Cl-C_6H_4$ | N | 159 - 162 |
| 90 | $4-F-C_6H_4$ | $2-Cl-C_6H_4$ | CH | 140 - 142 |
| 91 | $4-F-C_6H_4$ | $3-Cl-C_6H_4$ | N | |
| 92 | $4-F-C_6H_4$ | $4-Cl-C_6H_4$ | N | |
| 93 | $4-F-C_6H_4$ | $4-Cl-C_6H_4$ | CH | |
| 94 | $4-F-C_6H_4$ | $2,4-Cl_2-C_6H_3$ | N | 174 - 176 |
| 95 | $4-F-C_6H_4$ | $2,4-Cl_2-C_6H_3$ | CH | 155 - 157 |
| 96 | $4-F-C_6H_4$ | $2-F-C_6H_4$ | N | 134 - 139 |
| 97 | $4-F-C_6H_4$ | $2-F-C_6H_4$ | CH | 121 |
| 98 | $4-F-C_6H_4$ | $4-F-C_6H_4$ | CH | |
| 99 | $4-F-C_6H_4$ | $4-F-C_6H_4$ | N | |
| 100 | $4-F-C_6H_4$ | $4-NO_2-C_6H_4$ | N | |
| 101 | $4-F-C_6H_4$ | $3-NH_2-C_6H_4$ | N | |
| 102 | $4-F-C_6H_4$ | $2-OCH_3-C_6H_4$ | N | |
| 103 | $4-F-C_6H_4$ | Cyclohexyl | N | |
| 104 | $2,4-Cl_2-C_6H_3$ | Phenyl | N | |
| 105 | $2,4-Cl_2-C_6H_3$ | $2-Cl-C_6H_4$ | N | |
| 106 | $2,4-Cl_2-C_6H_3$ | $3-Cl-C_6H_4$ | N | |
| 107 | $2,4-Cl_2-C_6H_3$ | $4-Cl-C_6H_4$ | N | |
| 108 | $2,4-Cl_2-C_6H_3$ | $2,4-Cl_2-C_6H_3$ | N | |
| 109 | $2,4-Cl_2-C_6H_3$ | $2-F-C_6H_4$ | N | |
| 110 | $2,4-Cl_2-C_6H_3$ | $4-F-C_6H_4$ | N | |
| 111 | $2,4-Cl_2-C_6H_3$ | $4-NH_2-C_6H_4$ | N | |
| 112 | $2,4-Cl_2-C_6H_3$ | $2-OCH_3-C_6H_4$ | N | |
| 113 | $2,4-Cl_2-C_6H_3$ | Cyclohexyl | N | |
| 114 | $2-OCH_3-C_6H_4$ | $2-Cl-C_6H_4$ | N | |
| 115 | $2-OCH_3-C_6H_4$ | $4-Cl-C_6H_4$ | N | |
| 116 | $2-OCH_3-C_6H_4$ | $2-F-C_6H_4$ | N | |
| 117 | $2-OCH_3-C_6H_4$ | $4-F-C_6H_4$ | N | |
| 118 | $4-OCH_3-C_6H_4$ | $2-Cl-C_6H_4$ | N | Harz |
| 119 | $4-OCH_3-C_6H_4$ | $4-Cl-C_6H_4$ | N | |
| 120 | $4-OCH_3-C_6H_4$ | $2-F-C_6H_4$ | N | |
| 121 | $4-OCH_3-C_6H_4$ | $4-F-C_6H_4$ | N | |
| 122 | Cyclohexyl | $2-Cl-C_6H_4$ | N | |
| 123 | Cyclohexyl | $4-Cl-C_6H_4$ | N | |
| 124 | Cyclohexyl | $2-F-C_6H_4$ | N | |

| Beispiel | $R_1$ | $R_2$ | X | Schmp./IR (°C) |
|---|---|---|---|---|
| 125 | Cyclohexyl | $4-F-C_6H_4$ | N | |
| 126 | Cyclohexyl | $2,4-Cl_2-C_6H_3$ | N | |
| 127 | Cyclohexyl | $4-F-C_6H_4$ | CH | |
| 128 | Cyclohexyl | Cyclohexyl | N | |
| 129 | $4-F-C_6H_4$ | $2-CF_3-C_6H_4$ | N | 103-106 |
| 130 | $4-F-C_6H_4$ | $2-CF_3-C_6H_4$ | CH | 153 |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff

oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 2 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 7 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 34 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 34 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 39 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 89 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 1 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 2 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N′-propylen-bis-dithiocarbamat),
Zink-(N,N′-propylen-bis-dithiocarbamat),

N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,

DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiele

Als Vergleichswirkstoff wurde 1-(1,2,4-Triazol-1-ylmethyl)-1-(4-chlorphenyl)-3-(2-chlorphenyl)-prop-2-en-1-ol (A) - bekannt aus EP 52 424 - benutzt.

Anwendungsbeispiel 1

Wirksamkeit gegen Pyrenophora teres

Gerstenkeimlinge der Sorte "Igri" wurden im Zweiblattstadium mit wäßrigen Suspensionen, die 80 % (Gew.%) Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18 °C gestellt. Anschließend wurden die Pflanzen im Gewächshaus bei 20 - 22 °C und 70 % relativer Luftfeuchtigkeit für weitere 5 Tage kultiviert. Dann wurde das Ausmaß der Symptomentwicklung ermittelt.

Das Ergebnis zeigt, daß die Wirkstoffe 1, 2, 7, 34, 39 und 89 bei der Anwendung als 0,05 %ige Spritzbrühe eine bessere fungizide Wirkung (90 %) zeigen als der bekannte Vergleichswirkstoff A (50 %).

**Ansprüche**

1. Azolylmethylallylalkohole der allgemeinen Formel I

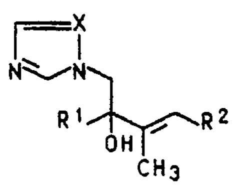

(I)

in welcher $R_1$ und $R_2$ gleich oder verschieden sind und $C_1$-$C_4$-Alkyl, Naphthyl, Biphenyl, $C_3$-$C_{12}$-Cycloalkyl, Cycloalkenyl oder Phenyl bedeuten, wobei diese Reste gegebenenfalls durch Halogen, Nitro, Phenoxy, Alkyl, Alkoxy, Amino oder Halogenalkyl mit jeweils 1 bis 4-C-Atomen substituiert sind,
X, CH oder N bedeutet, sowie deren für Pflanzen verträgliche Säureadditionssalze und Metallkomplexe.

2. Azolylmethylallylalkohole der Formel I, in denen $R_1$ einen unsubstituierten oder durch Fluor oder Chlor substituierten Phenylrest bedeutet.

3. Verfahren zur Herstellung der Azolylmethylallylalkohole der Formel I gemäß Anspruch 1, dadurch gekennzeichnet, daß man eine Verbindung der Formel II

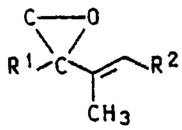

II

In welcher $R_1$, $R_2$ und X die oben angegebenen Bedeutungen haben mit einer Verbindung der Formel III

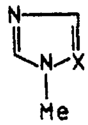

III

in der Me ein Wasserstoffatom oder ein Metallatom bedeutet und X die oben angegebene Bedeutung hat, zur Umsetzung bringt und die so erhaltenen Verbindungen gegebenenfalls in ihre für Pflanzen verträglichen Salze überführt.

4. Fungizides Mittel, enthaltend einen Azolylmethylallylalkohol der allgemeinen Formel I

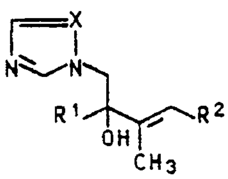

(I)

in welcher $R_1$ und $R_2$ gleich oder verschieden sind und $C_1$-$C_4$-Alkyl, Naphthyl, Biphenyl, $C_3$-$C_{12}$-Cycloalkyl, Cycloalkenyl oder Phenyl bedeuten, wobei diese Reste gegebenenfalls durch Halogen, Nitro, Phenoxy, Alkyl, Alkoxy, Amino oder Halogenalkyl mit jeweils 1 bis 4-C-Atomen substituiert sind,
X, CH oder N bedeutet, oder dessen für Pflanzen verträgliches Säureadditionssalz oder Metallkomplex.

5. Verfahren zur Bekämpfung von Pilzen, dadurch gekennzeichnet, daß man eine fungizid wirksame Menge eines Azolylmethylallylalkohols der allgemeinen Formel I

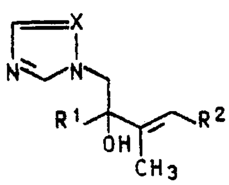

(I)

in welcher $R_1$ und $R_2$ gleich oder verschieden sind und $C_1$-$C_4$-Alkyl, Naphthyl, Biphenyl, $C_3$-$C_{12}$-Cycloalkyl, Cycloalkenyl oder Phenyl bedeuten, wobei diese Reste gegebenenfalls durch Halogen, Nitro, Phenoxy, Alkyl, Alkoxy, Amino oder Halogenalkyl mit jeweils 1 bis 4-C-Atomen substituiert sind,
X, CH oder N bedeutet, oder dessen für Pflanzen verträgliches Säureadditionssalz oder Metallkomplex, auf die Pilze oder auf durch Pilzbefall bedrohte Materialien, Flächen, Pflanzen oder Saatgüter einwirken läßt.

6. Verbindung gemäß Anspruch 1, dadurch gekennzeichnet, daß $R^1$ 4-Chlorphenyl, $R^2$ 2-Chlorphenyl und X den Rest N bedeutet.

7. Verbindung gemäß Anspruch 1, dadurch gekennzeichnet, daß $R^1$ 4-Chlorphenyl, $R^2$ 2-Chlorphenyl und X den Rest CH bedeutet.

8. Verbindung gemäß Anspruch 1, dadurch gekennzeichnet, daß $R^1$ Phenyl, $R^2$ 2,4-Dichlorphenyl und X den Rest N bedeutet.

9. Verbindung gemäß Anspruch 1, dadurch gekennzeichnet, daß $R^1$ 4-Fluorphenyl, $R^2$ 2-Chlorphenyl und X den Rest N bedeutet.